Europäisches Patentamt

European Patent Office

Office européen des brevets

(11) Publication number: **0 275 695 B1**

## (12) EUROPEAN PATENT SPECIFICATION

(45) Date of publication of patent specification: **30.09.92** (51) Int. Cl.⁵: **A61K 7/32**

(21) Application number: **87311312.0**

(22) Date of filing: **22.12.87**

---

(54) **Cosmetic product.**

---

(30) Priority: **23.12.86 GB 8630722**

(43) Date of publication of application:
**27.07.88 Bulletin  88/30**

(45) Publication of the grant of the patent:
**30.09.92 Bulletin  92/40**

(84) Designated Contracting States:
**AT BE CH DE ES FR GB GR IT LI NL SE**

(56) References cited:
**FR-A- 2 099 687
FR-A- 2 274 278
GB-A- 2 048 229
GB-A- 2 144 992
US-A- 4 508 705**

**INFORMATION ON SILICONE FLUIDS, Dow
Corning Corp., 1978**

(73) Proprietor: **UNILEVER PLC
Unilever House Blackfriars P.O. Box 68
London EC4P 4BO(GB)**

(84) Designated Contracting States:
**GB**

(73) Proprietor: **UNILEVER N.V.
Burgemeester s'Jacobplein 1 P.O. Box 760
NL-3000 DK Rotterdam(NL)**

(84) Designated Contracting States:
**BE CH DE ES FR GR IT LI NL SE AT**

(72) Inventor: **Park, Andrew Campbell
12 Dovepoint Road
Great Meols Hoylake Wirral L47 6AR(GB)**

(74) Representative: **Elliott, Peter William et al
Unilever plc, Patent Division Colworth House
Sharnbrook
Bedford MK44 1LO(GB)**

---

EP 0 275 695 B1

**Description**

FIELD OF INVENTION

The invention relates to propellant-containing antiperspirant products of the type comprising a finely divided antiperspirant active agent suspended in a liquid medium, which products have an exceptionally high efficacy in use. The invention is also concerned with a process for making these high efficacy antiperspirant products.

BACKGROUND AND PRIOR ART

For inhibiting perspiration, the application to the skin of many different antiperspirant active compounds has been described in the literature. However, those compounds most widely employed in commercial products of the aerosol type for this purpose are basic aluminium halides, especially aluminium chlorohydrate which has an aluminium/chlorine molar ratio of about 2. Especially popular are aerosol products in which the antiperspirant active is present in a finely divided form. These have been described as powder aerosols.

The effectiveness of an antiperspirant product can be assessed by subjecting human volunteers to thermal stress and gravimetric determination of axilla sweat.

By employing such a procedure, it can be shown that powder aerosol antiperspirant products are more effective in reducing or eliminating the appearance of sweat at the skin surface than the so-called solution type antiperspirants, as the powder form allows for a greater amount of the antiperspirant active to be delivered to and deposited on the skin surface. Even so, powder antiperspirant products currently available on the market generally have limited efficacy in reducing perspiration, as quantified and expressed in terms of percentage sweat reduction.

A further problem which is inherent in antiperspirant products of the powder aerosol type is the tendency for the finely-divided powder antiperspirant agent to settle in the aerosol container, particularly if the product is left undisturbed and not used for several days, or weeks, or even longer. The settled particles of antiperspirant active can form a hard cake at the bottom of the container, which can be difficult to break up and disperse, even with vigorous shaking; this can lead to poor dispensing properties or blockage of the nozzle or dip tube thus preventing further use of the product.

Just such a problem can occur with a powder aerosol antiperspirant product such as that described by Colgate-Palmolive Company in their South African patent specification 75/3576, by following the teaching of for instance Example 1 on page 10 which discloses a composition having the following ingredients:

| | % by weight |
|---|---|
| Aluminum basic chloride ($Al_2OH_5Cl$) | 3.0 |
| Anhydrous ethanol | 10.0 |
| 'Bentone 27' | 0.5 |
| Propylene carbonate | 0.165 |
| Isopropyl palmitate | 0.5 |
| Propellant dichlorodifluoromethane/ | |
| dichlorotetrafluoroethane (25:75) | 85.835 |

According to Colgate-Palmolive, their product is prepared by first making a suspension concentrate by blending the 'Bentone 27' suspending agent and the volatile solvent (alcohol). The propylene carbonate (wetting agent) is then added and the mixture vigorously mixed to form a gel. The remaining non-propellant ingredients, according to Colgate-Palmolive, are then added and the composition mixed to form a homogenous mixture. The concentrate is then placed in a can, sealed with a suitable aerosol valve, and pressurised with the propellant mixture.

Note that the weight ratio of the suspending agent to the alcohol is 1:20 (ie 0.05) in each of the three technical examples which illustrate the Colgate-Palmolive composition, and that there is no teaching in their

2

patent specification to illustrate the benefits and advantages of using other weight ratios of these two ingredients.

The efficacy of the Colgate Palmolive composition as set out above has been compared with that of the composition according to the invention. The experimental technique used and the results obtained are given in detail later in this specification. It is apparent that the weight ratio of the suspending agent to the alcohol has a critical influence on the efficacy of antiperspirant composition, and that this weight ratio as taught by Colgate-Palmolive is too low for optimum efficacy.

In GB 2,144,992-A, there is described a composition containing zirconyl hydroxychloride and aluminium chlorhydroxide having improved antiperspirant efficacy. This composition is including in various compositions for topical application to the skin, including a suspension in a liquified propellant for aerosol application, which additionally contains (by weight) 2% of talc, 4.2% of Anhydrous Alcohol SDA-40, 1.4% of quaternium-18 hectorite, 4.0% of cyclomethicone, 3.0% of isopropyl myristate, 0.1% of perfume, and 78.3% of Propellant-A 31.

According to GB-1 395 484 of The Procter & Gamble Company, a propellant-containing suspending agent, particularly for use with aerosol antiperspirant products, is prepared by subjecting to high shear mixing a combination of from 0.1 to 3.2% by weight of a clay bulking agent with from 0 to 0.45% parts by weight, per part by weight of the bulking agent of a polar organic liquid such as aqueous ethanol, with a balancing amount of an anhydrous liquefiable propellant. The propellant-containing suspending agent can then be used in order to suspend finely divided particulate material such as an antiperspirant agent.

Applicants' invention is based on the discovery that the selection of a much higher weight ratio of a specially chosen clay suspending agent to anhydrous alcohol provides a product having an unexpectedly greater efficacy in use which could not be deduced from the teaching of either Colage-Palmolive or Procter & Gamble.

Applicants have also discovered that by employing a special process in which high boiling propellant is sheared together with the specially chosen clay suspending agent before the addition of a finely divided antiperspirant agent and a low boiling propellant, the anhydrous alcoholic powder aerosol product so prepared not only shows superior antiperspirant efficacy, but it also does not suffer from the hard settling problem leading to caking of antiperspirant active within the aerosol container, which can occur with this type of antiperspirant product.

DEFINITION OF THE PRODUCT INVENTION

Accordingly, the invention provides an anhydrous propellant-containing antiperspirant product of the type comprising a finely-divided antiperspirant active agent suspended in a liquid phase, which product comprises:

a) from 0.5% to 20% by weight of a finely-divided powder antiperspirant agent having a maximum particle diameter of no greater than 100 $\mu$m which is substantially insoluble in the liquid phase of the product;

b) from 2 to 25% by weight of anhydrous alcohol containing less than 1% by weight of water and chosen from ethanol, isopropanol and mixtures thereof;

c) from 0.5% to 8% by weight of a suspending agent chosen from hydrophobically treated clays;

d) from 0 to 3% by weight of an emollient liquid which is less volatile than isopropanol;

e) from 0 to 70% by weight of anhydrous propellant which is in a liquid state at 20°C and 760mm Hg pressure, chosen from non-halogenated hydrocarbons, fluorocarbons, chlorofluorocarbons, methylene chloride and mixtures thereof; and

f) from 5 to 90% by weight of anhydrous propellant which is in a gaseous state at 20°C and 760mm Hg pressure, chosen from non-halogenated hydrocarbons, fluorocarbons, chlorofluorocarbons, dimethyl ether, carbon dioxide, nitrogen and non-halogenated hydrocarbons and mixtures thereof;

provided that the weight ratio of the suspending agent to the anhydrous alcohol in the product is from 2:1 to 1:12.

DISCLOSURE OF THE PRODUCT INVENTION

The antiperspirant agent

The product according to the invention comprises an antiperspirant agent in the form of a finely divided powder having a maximum particle diameter of no greater than 100$\mu$, preferably no greater than 70$\mu$ and most preferably about 45$\mu$. It is important not to exceed maximum particle diameter of about 100$\mu$ for this

ingredient in order to maintain a stable suspension of the anti-perspirant agent in the final product, thereby avoiding sedimentation or settling of this powder within the aerosol container with the consequent risk of nozzle blockage or poor in-use spray characteristics.

Examples of suitable antiperspirants in powder form, which are relatively insoluble or only slightly soluble in the anhydrous alcohol and the propellants employed, are aluminium chloride; aluminium sulphate; aluminium chlorohydrate; basic aluminium bromide, basic aluminium chloride glycine complexes, basic aluminium chloride urea complexes and aluminium zirconium chlorohydrates. Other preferred antiperspirant active materials of high efficacy are the special active forms of basic aluminium chloride which have a particular distribution of polymeric species in aqueous solution and obtainable by procedures described in US Patent No. 4 359 456 (Gosling et al). Similar processes for making highly active forms of aluminium chlorohydrate involving the ageing of aluminium chlorohydrate in an aqueous medium are described in British Patent Specification No 2 048 229 (Gillette). Other suitable materials are described in British Patent Specification No. 2 144 992 (Gillette). The antiperspirant agent may also be a urea or glycine complex of the higher active forms of aluminium chlorohydrate prepared as described in European Patent No. 6738 (Unilever) and British Patent No. 1 597 497 (Unilever), respectively.

Further examples of highly active aluminium chlorohydrate which can be employed as the antiperspirant agent are available from Reheis, under the trade name of REACH.

It is to be understood that the foregoing antiperspirant agents do not form an exhaustive list, and that the composition according to the invention can comprise a single antiperspirant agent or mixtures thereof.

The amount of the antiperspirant agent present in the product according to the invention is generally from 0.5 to 20%, preferably from 1 to 15% and most preferably from 2 to 12% by weight.

If an amount of less than 0.5% by weight of the antiperspirant agent is employed, then the efficacy of the product in use expressed in terms of sweat reduction (as hereinafter defined) is likely to be poor. Also, if an amount of greater than 20% by weight of the antiperspirant active is employed, then nozzle blockage can occur in use and the overall efficacy of the product is unlikely to be enhanced beyond that obtainable with products containing an amount of antiperspirant agent which falls within the range of from 0.5 to 20% by weight as herein defined.

The alcohol

The product according to the invention also comprises an alcohol chosen from anhydrous ethanol, anhydrous isopropanol and mixtures thereof. By "anhydrous" is meant that the alcohol should contain less than 1% by weight of water.

Use of aqueous alcohol, that is alcohol containing more than 1% by weight of water, can result in a product showing poor efficacy in use. Furthermore, when aluminium or tin-plate containers are used to contain such an aqueous alcoholic product, then corrosion of the metal can occur. It is accordingly for the above reasons important to select anhydrous alcohol for the product of the invention.

The amount of alcohol present in the product according to the invention is from 2 to 25%, preferably from 5 to 20% by weight.

If an amount of less than 2% by weight of the alcohol is employed, then the efficacy of the final product expressed in terms of sweat reduction (as hereinafter defined) can be low. If an amount of greater than 25% by weight of the alcohol is employed, then the spray properties of the final product can be impaired and its efficacy in use can also be low.

Suspending agent

The product according to the invention also comprises a suspending agent which is chosen from hydrophobically treated clays.

A preferred class of hydrophobically-treated clays comprise smectite clays, examples of which include the montmorillonites, hectorites, and colloidal magnesium aluminium silicates.

Montmorillonite is colloidal, hydrated aluminium silicate obtained from bentonite of which it is the predominant constituent. A detailed discussion of bentonites can be found in the Kirk-Othmer Encyclopedia of Chemical Technology, 2nd Edition, Vol. 3 (1964) pp. 339-360, published by Interscience Publishers.

Hectorite, also a smectite clay, differs from montmorillonite in that there is almost a complete substitution of aluminium in the lattice structure of montmorillonite by magnesium and in addition, it contains lithium and fluorine.

The magnesium aluminium silicates are complexes of colloidal magnesium aluminium silicate richer in magnesium than aluminium. The hydrophobically treated magnesium aluminium silicates are commercially

4

available under the name Veegum PRO from the R T Vanderbilt Co.

Preferred suspending agents for use in the present invention are hydrophobic clays available under the trade name of "Bentone". Bentones are prepared by reacting a suitable clay in a cation exchange system with an amine. Different amines are reacted to obtain a variety of Bentones, which may also differ in proportions of Si, MgO and $Al_2O_3$. Examples of useful "Bentone" suspending agents are "Bentone-27", which is a stearaluminium hectorite; "Bentone-34", which is quaternium 18 bentonite; "Bentone-38", which is quaternium 18 hectorite; and "Bentone-14" which is a clay-extended quaternium 18 hectorite, all of which have a particle size of below 5 microns and are commercially available from NL Industries Inc. Other suitable "Bentone" clays are Bentone SD1 and Bentone SD2.

Yet further suitable clays are those hydrophobically treated smectite clays available under the trade names Perchem and Tixogel. Specific examples are Perchem clays 44, 97 and 108 and Tixogel VZ.

A further class of hydrophobically treated clays comprises hormite clays, an example of which is hydrophobically modified attapulgite clay available under the name Perchem DMA. Perchem clays are sold by Perchem Limited of Harlow, Essex, Great Britain and Tixogel clays by Production Chemicals Limited of Stockport, Cheshire, Great Britain.

The amount of suspending agent present in the product according to the invention is from 0.5 to 8%, preferably from 1 to 5% by weight.

If an amount of less than 0.5% by weight of the suspending agent is employed, then the suspending properties of the final product are likely to be poor. Also, the efficacy of the final product expressed in terms of sweat reduction (as hereinafter defined) can be low. If an amount greater than 8% by weight of the suspending agent is employed, then the viscosity of the final product can be too high in that poor spray characteristics or valve blockage can result.

## Weight ratio of suspending agent to alcohol

It is preferred that the weight ratio of the suspending agent to the alcohol is from 2:1 to 1:12 (ie 2 to 0.08), preferably from 1:1 to 1:10 (ie 1 to 0.1) and most preferably from 1:1 to 1:7 (ie 1 to 0.14).

It is apparent that if the weight ratio of the suspending agent to the alcohol falls outside the broadest range as herein defined, that is, >2 or <0.08, then the efficacy of the product is likely to be no better than the best of the commercially available products known to Applicants, and/or there will be a tendency for the powder antiperspirant agent to settle in the aerosol container when not in use, and there form a 'cake' which is difficult to break up by shaking the container.

## The emollient liquid

The product according to the invention can also comprise an additional liquid which is less volatile than isopropanol. Such additional liquids can be incorporated to give emollient cosmetic benefits; they can be either hydrophilic or hydrophobic liquids.

Examples of suitable emollient liquids include polar liquids such as water-miscible polyoxyalkylene glycol or a water-miscible partial butyl ether thereof; hexylene glycol; a $C_1$-$C_4$ alkyl monoether of a simple or condensed $C_2$-$C_4$ alkylene glycol, for example dipropyleneglycol monomethyl ether; and 2-ethyl-1,3-hexane diol.

Examples of other commercially available hydrophilic emollient liquids are Pluronics (eg. Poloxamer 101, Poloxamer 105, Poloxamer 181, Poloxamer 182), Carbowaxes (eg. PEG-4, PEG-8, PEG-12), Witconol APEM (PPG-3 Myreth-3), Witconol APES (PPG-9 Steareth-3), Standamul OXL (PPG-10 Ceteth-20), Procetyl AWS Modified (PPG-8 Ceteth-2), glycols and their citrate, lactate and tartrate esters. Particularly preferred hydrophilic emollient liquids which are especially useful are the dimethicone copolyols which are polymers of dimethylsiloxane with polyoxyethylene and/or polyoxypropylene side chains. Examples of these are SILWET L-720, L-7600, and L7610 from Union Carbide, Silicone 190 and 193 surfactants both from Dow Corning and ABIL B 8842, 8843 and 8851 from Goldschmidt. These dimethicone copolyols are also referred to in the literature as polyalkylene oxide modified dimethylpolysiloxanes, as silicone glycol copolymers and as polysilicone polyether copolymers. Dimethyl isosorbide is a further example of a suitable hydrophilic emollient liquid.

Examples of non-polar water-immiscible emollient liquids that may be present in minor proportion are the linear volatile silicones having 2 to 9 silicone atoms such as hexamethyldisiloxane, the cyclic volatile silicones having 3 to 6 silicone atoms such as the tetramer and pentamer and mixtures thereof, paraffin oils, fatty acid esters such as isopropyl myristate and dibutyl phthalate, and polyoxypropylene fatty ethers such as Fluid AP.

The amount of emollient liquid which is less volatile than ethanol or isopropanol that can be present in the product according to the invention is from 0 to 3%, preferably from 0.5 to 3% by weight.

The liquid propellant

The product according to the invention also preferably comprises an anhydrous propellant which is in a liquid state at 20°C and 760mm Hg pressure, hereinafter referred to as the 'liquid propellant', which is chosen from non-halogenated hydrocarbons, fluorocarbons, chlorofluorocarbons, methylene chloride and mixtures thereof.

Examples of liquid propellants include pentane, isopentane, hexane, Propellants 11 (trichlorofluoromethane) and 113 (trichlorotrifluoroethane).

The amount of liquid propellant present in the product according to the invention is from 0 to 70%, preferably from 5 to 70%, and ideally from 20 to 60% by weight, expressed in terms of the final product.

If an amount of liquid propellant is above 70%, then the efficacy and the suspending properties of the final product can be impaired.

The gaseous propellant

The product according to the invention also comprises an anhydrous propellant which is in a gaseous state at 20°C and 760 mm Hg pressure, hereinafter referred to as the 'gaseous propellant', which is chosen from dimethylether, carbon dioxide, nitrogen, non-halogenated hydrocarbons, fluorocarbons, chlorofluorocarbons and mixtures thereof.

Examples of gaseous propellants include propane, n-butane, isobutane, Propellants 21 (dichlorofluoromethane), 114 (dichlorotetrafluoroethane), C-318 (octafluorocyclobutane), 1141 (monofluoroethane) 22 (chlorodifluoromethane), 115 (chloropentafluoroethane), 12 (dichlorodifluoromethane), 152a (difluoroethane) and 142b (chlorodifluoroethane).

The amount of gaseous propellant present in the product according to the invention is from 5 to 90%, preferably from 20 to 80% and ideally from 40 to 60% by weight, expressed in terms of the final product.

If an amount of gaseous propellant outside the widest of the above defined ranges is employed, then the efficacy and suspending properties of the final product can be impaired.

Perfume

The product according to the invention can also optionally comprise a perfume. If so, then it should be chosen from any suitable perfume that is compatible with the other ingredients present in the final product and it should also be acceptable to the consumer. A particularly preferred perfume is a deodorant perfume such as one described in GB 2 013 493 (Unilever Limited).

The amount of perfume when employed in the process according to the invention is preferably from 0.001 to 2%, most preferably from 0.1 to 1% by weight.

Other antiperspirant adjuncts

The product according to this invention can also optionally comprise antiperspirant adjuncts other than the materials already referred to in the specification, in order to enhance or improve the properties of the final product. Examples of other antiperspirant adjuncts can include antimicrobial agents such as IRGASAN DP 300, a corrosion inhibitor such as sodium benzoate, aluminium hydroxide and urea, or a drying agent such as Molecular Sieve Type 3A to reduce or eliminate the possibility of corrosion occurring when an aluminium or tin-plate can is employed.

The amount by weight of other antiperspirant adjuncts which can optionally be present in the product according to the invention will generally form the balance of the final product.

DEFINITION OF THE PROCESS INVENTION

The invention also provides a process for preparing an anhydrous propellant-containing antiperspirant product of the type comprising a finely-divided antiperspirant active suspended in a liquid phase, which process comprises the steps of:
(i) combining in a suitable container:
(a) from 0.5 to 8% by weight, expressed in terms of the final product, of a suspending agent chosen

6

from hydrophobically treated smectite clays;

(b) an amount of from 0.5 to 25% by weight, expressed in terms of the final product, of anhydrous alcohol containing less than 1% by weight of water and chosen from ethanol, isopropanol and mixtures thereof; and

(c) from 5 to 70% by weight, expressed in terms of the final product, of anhydrous propellant which is a liquid at 20°C and 760 mm Hg pressure, chosen from non-halogenated hydrocarbons, fluorohydrocarbons, chlorofluorohydrocarbons, methylene chloride and mixtures thereof;

(ii) Subjecting the combination to high shear mixing for a period of time sufficient to achieve a uniform dispersion of the suspending agent; and

(iii) mixing the dispersion with:

(a) from 0.5 to 20% by weight, expressed in terms of the final product, of a finely-divided powder antiperspirant agent having a maximum particle diameter of no greater than 100 $\mu$m which is substantially insoluble in the liquid phase of the product, and

(b) from 5 to 90% by weight, expressed in terms of the final product, of anhydrous gaseous propellant, chosen from non-halogenated hydrocarbons, fluorocarbons, chlorofluorocarbons, dimethyl ether, carbon dioxide, nitrogen and mixtures thereof;

(c) a further quantity if necessary of the alcohol such that the final product contains from 2 to 25% by weight of alcohol;

to form the antiperspirant product.

## DISCLOSURE OF THE PROCESS INVENTION

The process for preparing the propellant-containing antiperspirant product of the invention accordingly comprises three fundamental steps.

In the first step of the process, from 0.5 to 8% by weight, expressed in terms of the final product, of the suspending agent, from 0 to 25% preferably from 0.5 to 20% by weight, expressed in terms of the final product, of the anhydrous alcohol, and from 0 to 70%, preferably 5 to 70% by weight, expressed in terms of the final product, of the liquid propellant are combined in a suitable container. Choice of a suitable container will be governed by such factors as batch size, and whether or not cooling, heating and super-atmospheric pressures are required.

In the second step of the process, the combination is subjected to high shear mixing for a period of time sufficient to achieve a uniform dispersion of the suspending agent. High shear mixing is necessary to effect maximum dispersion of the suspending agent in the alcohol and propellant, which cannot be achieved utilising non-shear mixing methods. The concept of shear mixing is well known and a discussion of it can be found in the literature, for example Chemical Engineering, Volume 2, second edition by Coulson and Richardson. The term 'high shear mixing' relates to a degree of shear mixing which can be accomplished using a number of well known means. The degree of high shear mixing is not critical to the present invention and the products of the invention can readily be achieved using a variety of equipment having a range of shear mixing capacities. Examples of suitable high shear mixing equipment are shear pumps, colloid mills, shear-in-line mixers, dispensators and blenders for small scale formulation. Shear mixing should be continued until the uniform dispersion of the suspending agent is obtained the point of maximum dispersion is achieved when a substantially constant viscosity is reached.

In the third step of the process, the dispersion obtained following high shear mixing is then mixed with from 0.5 to 20% by weight, expressed in terms of the final product, of the finely-divided (powdered) antiperspirant agent, from 5 to 90% by weight, expressed in terms of the final product of the anhydrous gaseous propellant to form the antiperspirant product which can also comprise perfume and other optional antiperspirant adjuncts including an emollient liquid less volatile than ethanol and isopropanol, to form the balance of the product.

In a preferred process according to the invention, the third step of the process comprises two parts. Firstly, the dispersion derived from the second step is mixed with the antiperspirant agent and filled into aerosol containers, and secondly, the containers are fitted with valves and the gaseous propellant is then introduced into the containers to the desired pressure.

The antiperspirant product so obtained can be packaged to the desired pressure in a conventional aerosol container of glass, plastics material or metal, such as tin plate or aluminium which is preferably lacquered on the inside to reduce or eliminate corrosion, the container being fitted with a dispensing valve for actuation to deliver the product as and when required by the user.

## ANTIPERSPIRANT PRODUCT EFFICACY

It is a feature of the antiperspirant products of the invention that when used to control perspiration, for example in the axilla, then the appearance of perspiration at the skin surface can be reduced by an amount of up to 60% or more of the perspiration generated without the protection of an antiperspirant product. Having regard to the fact that the best of the commercially available antiperspirant products known to us are unable to reduce the appearance of perspiration at the skin surface by more than about 45%, then it is evident that we have made a substantial advance in the art of perspiration control. The effect that we are able to demonstrate is entirely surprising and unexpected, and the mechanism cannot adequately be explained, although it is apparent that the selection of a hydrophobically treated smectite clay as the suspending agent, and anhydrous alcohol is all important. It is also apparent that the choice of the weight ratio of suspending agent to alcohol present in the product, as herein defined, is critical to obtaining a high degree of efficacy in use.

Accordingly, it is a property of the antiperspirant product of the invention that on application to human skin, particularly to the axilla, a higher degree of perspiration control can be obtained than with any currently available products. It is furthermore apparent that irrespective of the choice of antiperspirant agent, its efficacy as expressed in terms of its ability to reduce sweat production is enhanced beyond all expectations when employed in a product according to the invention.

MEASUREMENT OF EFFICACY OF THE ANTIPERSPIRANT PRODUCT

In the following part of this specification references are made to the antiperspirant efficacy of various products. Before giving details of the composition of these products the test procedure carried out to evaluate their antiperspirant efficacy will first be described. The test procedure, which involves subjecting human volunteers to thermal stress and gravimetric determination of axilla sweat, is summarised as follows:

| | |
|---|---|
| Subjects: | A panel of up to 60 women who use no antiperspirant for the 14 days before the test. |
| Hot Room: | Temperatures $40°C \pm 2°C$; relative humidity $40\% \pm 5\%$. |
| Products: | When testing two products, one being designated the test product and the other the control, the panel is divided into two equal groups. One group receives the test treatment on the left axilla and the control treatment on the right, while the second group receives them the other way round. |
| | Alternatively, when comparing two test products against each other and against a control product, then the products are randomly applied to the axillae of the panel subjects, with the proviso that the product applied to the left axilla is different from that applied to the right axilla of each subject. |
| Control Product: | This is a placebo deodorant in the form of an aerosol product comprising by weight 25% ethanol, 0.6% isopropyl myristate, 0.3% perfume, and 74.1% propellant (1:1 mixture of Propellants 11 & 12). |
| Product Application: | The operator conducting the test applies the test product in a standard manner, that is by spraying at the axilla for approximately 2 seconds from a distance of approximately 15cm. |
| Sweat Collection: | Absorbant cotton pads are used to collect the sweat. On entering the hot room each panellist is subjected to a 40 minute 'warm-up' period, during which no sweat is collected. Sweat is then collected for a 20 minutes period and sweat weight determined. |
| Test Design: | Subjects attend daily for 3 consecutive days. They receive one treatment with the products on each of the first three days. Following product application on the third day, the panellist is subjected to a hot room sitting and sweat is collected. |
| Analysis of Data: | The statistical treatment includes an analysis of variance which allows for side effects due to the product and the panellist. The efficacy is calculated from the geometric mean weight of sweat collected from the axillae treated with each product using the formula: |

$$\% \text{ sweat reduction} = 100 \frac{(C-T)}{C}$$

where C is the geometric means sweat weight from the axillae treated with the control product and T is the geometric mean sweat weight from the axillae treated with the test product where a correction has been made for the side effect.

Significance is calculated by applying Student's t-test to the logarithmically transformed weights.

A COMPARISON OF THE ANTIPERSPIRANT EFFICACY OF THE COLGATE-PALMOLIVE COMPOSITION WITH A CORRESPONDING PRODUCT ACCORDING TO THE INVENTION

A comparison of the efficacy of the composition taken from the Colgate-Palmolive Company's South African patent specification No. 75/3576, as set out earlier in this specification, was compared with a corresponding product according to the invention using the panel of human volunteers as described hereinbefore.

For reasons of safety, certain changes were made to each formula, namely that Bentone 38 was used in place of Bentone 27 as the suspending agent. Also, the antiperspirant agent chosen was activated aluminium chlorohydrate in place of aluminium basic chloride, and the normally liquid propellant was trichlorofluoromethane in place of trichlorotetrafluoromethane, in view of non-availability of the latter. The remaining differences between the two formulations reflect the criteria essential to the present invention which are not found in the Colgate-Palmolive composition.

The formulation of the product according to the invention (A) and that of Colgate-Palmolive (B) were as follows:-

|  | % w/w | |
| --- | --- | --- |
|  | A | B |
| Activated aluminium chlorohydrate | 3 | 3 |
| Anhydrous ethanol (RR Grade) | 7.5 | 10 |
| Bentone 38 (suspending agent) | 2 | 0.5 |
| Isopropyl palmitate | – | 0.5 |
| Propylene carbonate | – | 0.165 |
| Perfume | 0.5 | – |
| Propellant | | |
| Cap 30 | 33 | |
| Trichlorofluoromethane (Arcton 11) | 35 | 55.795 |
| Dichlorodifluoromethane (Arcton 12) | 19 | 30.04 |
| Weight ratio of suspending agent to alcohol | 0.27 | 0.05 |

RESULTS

Following testing by the panel of human volunteers according to the regime described hereinbefore, it was found that the product according to the invention produced a 14% greater sweat weight reduction that that of Colgate-Palmolive. This indicates that the product of the invention has an antiperspirant efficacy which is significantly superior to that obtainable from the closest prior act reference.

Experiment to illustrate the importance of the presence of both a suspending agent and alcohol in the product according to the invention.

As has already been stated, it is an important feature of the invention that a suspending agent and alcohol should be present in the product according to the invention at a weight ratio of from 2:1 to 1:12 (ie from 2 to 0.08).

Formulations in which either a suspending agent or alcohol are omitted were prepared and tested by the panel of human volunteers to show that only poor efficacy scores, expressed in terms of percent sweat weight reduction, could be obtained.

The formulations which do not conform with the definition of the invention were as follows:

|  | % w/w | | | |
|---|---|---|---|---|
|  | C | D | E | F |
| Activated aluminium chlorohydrate | 4 | 4 | 10 | 5 |
| Anhydrous ethanol (RR grade) | 6 | - | - | - |
| Volatile silicone | - | - | 13.5 | 8 |
| Isopropyl myristate | - | 4.05 | - | - |
| Bentone 38 | - | 0.45 | 1 | 1.5 |
| Perfume | 0.5 | 0.44 | 1 | 0.5 |
| CAP 30 | - | - | 74.5 | - |
| Arcton 11 | 53.7 | 54.64 | - | 51 |
| Arcton 12 | 35.8 | 36.42 | - | 34 |
| Weight ratio of suspending agent to alcohol | 0 | ∞ | ∞ | ∞ |
| Efficacy (%SWR) | 36 | 38 | 35 | 45 |

The efficacy of each of these formulations was poor, thus indicating the necessity to include both a suspending agent and alcohol, at the specified weight ratios, in addition obtain superior efficacy which is a property of products according to the invention.

It should be noted that some of the trade names used in this specification, such as Bentone, Reach, Veegum PRO, Perchem, Tixogel, Poloxamer, Irgasan DP300, Arcton, and others may be Registered Trademarks in some of the Contracting States.

EXAMPLES

The invention is illustrated by the following examples.

Example 1

This example illustrates a product according to the invention and a process for preparing the product.

The Process

In this example, anhydrous ethanol (RR grade), the suspending agent Bentone 38 and the liquid propellant Arcton 11 (trichlorofluoromethane), where combined in a container and subjected to high shear mixing in an Ultra Turax T45 mixer fitted with a 45N shaft and a T45/6G generator, until a viscosity of about 1,000 cps was obtained: this is Reading 10 on Brookfield RTV 100 using A-Spindle at 20 rpm in the helical mode. It was necessary to top up with further Arcton 11 to take account of evaporation of this propellant.

A special active form of aluminium chloride and a perfume were then added to the dispersion obtained following high shear mixing, and after thorough mixing the mixture was filled into aerosol cans and then gassed with gaseous propellant Arcton 12 (dichlorodi-fluoromethane) according to conventional aerosol filling techniques.

The product

The product so obtained had the following composition:

| Ingredient | % by weight |
|---|---|
| AACH[1] | 4 |
| Anhydrous ethanol (RR grade) | 6 |
| Bentone 38 | 1.5 |
| Perfume | 0.5 |
| Arcton 11 | 52.8 |
| Arcton 12 | 35.2 |
| | 100 |

Weight ratio of suspending agent to alcohol is 0.25

1 - Spray dried powder of a special active form of aluminium chloride prepared according to US Patent No. 4 359 456. It had a particle size of < 75µ. It has a Band III percent aluminium value of great than 20%.

Efficacy

The efficacy of the product prepared by the process according to the invention was compared with that of a commercially available antiperspirant aerosol product.

The result showed that the product prepared by the process according to the invention had a sweat weight reduction value of 57%, whereas the commercially available product had a sweat weight reduction of 33%, clearly indicating the superiority of the product according to the invention.

Storage stability

The product prepared by the process according to the invention was stored at 0°, 28°, 37° and 45°C for 4 months. No settling problem to form a cake of the antiperspirant active at the bottom of the containers was observed in any of the samples. There was also no nozzle blockage problem.

Examples 2 to 4

The following examples illustrate further antiperspirant aerosol products according to the invention. In each case, the product was prepared and tested as described for Example 1, the normally gaseous propellants being a combination of Arcton 12 and CAP 30 in these examples.

The following table summaries the formulation of each product, together with the efficacy value (expressed as percentage sweat reduction) determined in each case.

| Ingredient | Example: | percentage by weight | | |
|---|---|---|---|---|
| | | 2 | 3 | 4 |
| AACH | | 5 | 3 | 5 |
| Anhydrous ethanol (RR grade) | | 7.5 | 7.5 | 7.5 |
| Bentone 38 | | 2 | 2 | 3 |
| Perfume | | 0.5 | 0.5 | 0.5 |
| Cap 30* | | 31 | 33 | 32 |
| Arcton 11 | | 35 | 36 | 34 |
| Arcton 12 | | <u>19</u> | <u>19</u> | <u>18</u> |
| Weight ratio of suspending agent to alcohol | | 0.27 | 0.27 | 0.4 |
| Efficacy (%SWR) | | 61 | 54 | 50 |

* a mixture of n-propane, n-butane and isobutane designed to produce a pressure of 207kPa.

Example 5 to 9

The following examples illustrate further antiperspirant aerosol products according to the invention. In each case, the product was prepared and tested as described for Example 1.

The following table summarises the formulation of each product, together with the efficacy value (expressed as percentage sweat reduction), determined in each case.

12

Percentage by weight

| Ingredient | Example: 5 | 6 | 7 | 8 | 9 |
|---|---|---|---|---|---|
| A A C H | 4 | 10 | 10 | 5 | 5 |
| Anhydrous ethanol (RR Grade) | 6 | 10 | 15 | 7.5 | 7.5 |
| Bentone 38 | 0.5 | 4 | 4 | 1.5 | 2.5 |
| Perfume | 0.5 | 1 | 1 | 0.5 | 0.5 |
| Cap 30 | – | 75 | 70 | 31.5 | 31 |
| Arcton 11 | 53.4 | – | – | 35 | 34.5 |
| Arcton 12 | 35.6 | – | – | 19 | 19 |
| Weight ratio of suspending agent to alcohol | 0.08 | 0.4 | 0.27 | 0.2 | 0.33 |
| Efficacy (% SWR) | 48 | 58 | 50 | 55 | 57 |

## Claims

1. An anhydrous propellant-containing antiperspirant product suitable for topical application to the skin, which comprises:

a) from 0.5 to 20% by weight of a finely-divided powder antiperspirant agent having a maximum particle diameter of no greater than 100 $\mu$m which is substantially insoluble in the liquid phase of the product;

b) from 2 to 25% by weight of anhydrous alcohol containing less than 1% by weight of water and chosen from ethanol, isopropanol or mixtures thereof;

c) from 0.5 to 8% by weight of a suspending agent chosen from hydrophobically treated clays;

d) from 0 to 3% by weight of an emollient liquid which is less volatile than isopropanol;

e) from 0 to 70% by weight of anhydrous propellant which is in a liquid state at 20°C and 760mm Hg pressure, chosen from non-halogenated hydrocarbons, fluorocarbons, chlorofluorocarbons, methylene chloride and mixtures thereof; and

f) from 5 to 90% by weight of anhydrous propellant which is in a gaseous state at 20°C and 760mm Hg pressure, chosen from non-halogenated hydrocarbons, fluorocarbons, chlorofluorocarbons, dimethyl ether, carbon dioxide, nitrogen and mixtures thereof;

provided that the weight ratio of the suspending agent to the anhydrous alcohol in the product is from 2:1 to 1:12.

2. A product according to claim 1, in which the antiperspirant agent is an aluminium-containing basic halide.

3. A product according to claim 1 or 2, in which the antiperspirant agent is chosen from aluminium chloride, aluminium sulphate, aluminium chlorohydrate, basic aluminium chloride glycine complexes, basic aluminium chloride urea complexes, or mixtures thereof.

4. A product according to Claim 1, 2, or 3 in which the antiperspirant agent is chosen from urea or glycine complexes of aluminium chlorohydrate, or mixtures thereof.

5. A product according to any preceding claim, in which the antiperspirant agent forms from 1 to 15% by

EP 0 275 695 B1

weight.

6. A product according to any preceding claim in which the antiperspirant agent forms from 2 to 12% by weight.

7. A product according to any preceding claim, in which the alcohol forms from 5 to 20% by weight.

8. A product according to any preceding claim, in which the clay is a smectite clay.

9. A product according to any preceding claim in which the suspending agent forms from 1 to 5% by weight.

10. A product according to any preceding claim, in which the weight ratio of the suspending agent to alcohol is from 1:1 to 1:10.

11. A product according to any preceding claim, which comprises from 0.5 to 3% by weight of an emollient liquid which is less volatile than isopropanol.

12. A product according to any preceding claim, in which the liquid propellant is a hydrocarbon chosen from pentane, isopentane, and hexane or mixtures thereof.

13. A product according to any of claims 1 to 11, in which the liquid propellant is chosen from Propellants 11, 113, or mixtures thereof.

14. A product according to any preceding claim, in which the liquid propellant forms from 10 to 60% by weight.

15. A product according to any preceding claims in which the gaseous propellant is a hydrocarbon chosen from propane, isobutane and n-butane or mixtures thereof.

16. A product according to any of claims 1 to 14 in which the gaseous propellant is a fluorocarbon chosen from Propellant C-318, Propellant 1141, Propellant 21 or Propellant 114, or mixtures thereof.

17. A product according to any of claims 1 to 14 in which the gaseous propellant is a chlorofluorocarbon chosen from Propellants 22, 115, 12, 152a, 142b, or mixtures thereof.

18. A product according to any preceding claim, in which the gaseous propellant forms from 20 to 80% by weight.

19. A product according to any preceding claim, which additionally comprises from 0.001 to 2% by weight of a perfume.

20. A process for preparing an anhydrous propellant-containing antiperspirant product of the type comprising a finely-divided antiperspirant active suspended in a liquid phase according to Claim 1, which process comprises the steps of:
    (i) combining in a suitable container:
        (a) from 0.5 to 8% by weight, expressed in terms of the final product, of a suspending agent chosen from hydrophobically treated smectite clays;
        (b) from 0.5 to 25% by weight, expressed in terms of the final product, of an anhydrous alcohol containing less than 1% by weight of water and chosen from ethanol, isopropanol or mixtures thereof; and
        (c) from 5 to 70% by weight, expressed in terms of the final product, of anhydrous propellant which is a liquid at 20°C and 760mm Hg pressure, chosen from non-halogenated hydrocarbons, fluorocarbons, chlorofluorocarbons, methylene chloride and mixtures thereof;
    (ii) subjecting the combination to high shear mixing for a period of time sufficient to achieve a uniform dispersion of the suspending agent; and
    (iii) mixing the dispersion with:
        (a) from 0.5 to 20% by weight, expressed in terms of the final product, of a finely-divided powder

14

antiperspirant agent having a maximum particle diameter of no greater than 100 μm which is substantially insoluble in the liquid phase of the product, and

(b) from 5 to 90% by weight, expressed in terms of the final product, of anhydrous gaseous propellant chosen from non-halogenated hydrocarbons, fluorocarbons, chlorofluorocarbons dimethyl ether, cabon dioxide, nitrogen or mixtures thereof;

(c) a further quantity if necessary of the alcohol such that the final product contains from 2 to 25% by weight of alcohol;

to form the antiperspirant product.

**Patentansprüche**

1. Wasserfreies, Treibmittel enthaltendes, schweißhemmendes Produkt, geeignet zur äußeren Anwendung auf der Haut, das umfaßt:

a) 0,5 bis 20 Gew.-% eines feinzerteilten, pulvrigen, schweißhemmenden Wirkstoffes mit einem maximalen Teilchendurchmesser von nicht größer als 100 μm, der im wesentlichen unlöslich in der flüssigen Phase des Produkts ist;

b) 2 bis 25 Gew.-% eines wasserfreien Alkohols, der weniger als 1 Gew.-% Wasser enthält und ausgewählt ist aus Ethanol, Isopropanol oder Gemischen davon;

c) 0,5 bis 8 Gew.-% eines Suspensionsmittels, ausgewählt aus hydrophob behandelten Tonen;

d) von 0 bis 3 Gew.-% einer beruhigenden Flüssigkeit, die weniger flüchtig ist als Isopropanol;

e) 0 bis 70 Gew.-% eines wasserfreien Treibmittels, das bei 20°C und einem Druck von 760 mm Hg im flüssigen Zustand ist, ausgewählt aus nichthalogenierten Kohlenwasserstoffen, Fluorkohlenstoffen, Chlorfluorkohlenstoffen, Methylenchlorid und Gemischen davon; und

f) 5 bis 90 Gew.-% eines wasserfreien Teibmittels, das bei 20°C und einem Druck von 760 mm Hg im gasförmigen Zustand ist, ausgewählt aus nichthalogenierten Kohlenwasserstoffen, Fluorkohlenstoffen, Chlorfluorkohlenstoffen, Dimethylether, Kohlendioxid, Stickstoff und Gemischen davon;

vorausgesetzt, daß das Gewichtsverhältnis von Suspensionsmittel zu wasserfreiem Alkohol im Produkt 2:1 bis 1:12 beträgt.

2. Produkt nach Anspruch 1, worin das schweißhemmende Mittel ein aluminiumhaltiges basisches Halogenid ist.

3. Produkt nach Anspruch 1 oder 2, worin das schweißhemmende Mittel ausgewählt ist aus Aluminiumchlorid, Aluminiumsulfat, Aluminiumchlorhydrat, basischen Aluminiumchloridglycinkomplexen, basischen Aluminiumchloridharnstoffkomplexen oder Gemischen davon.

4. Produkt nach Anspruch 1, 2 oder 3, worin das schweißhemmende Mittel ausgewählt ist aus Harnstoff- oder Glycinkomplexen von Aluminiumchlorhydrat oder Gemischen davon.

5. Produkt nach einem der vorhergehenden Ansprüche, worin das schweißhemmende Mittel 1 bis 15 % des Gewichts beträgt.

6. Produkt nach einem der vorhergehenden Ansprüche, worin das schweißhemmende Mittel 2 bis 12 % des Gewichts beträgt.

7. Produkt nach einem der vorhergehenden Ansprüche, worin der Alkohol 5 bis 20 % des Gewichts beträgt.

8. Produkt nach einem der vorhergehenden Ansprüche, worin der Ton ein Smektit-Ton ist.

9. Produkt nach einem der vorhergehenden Ansprüche, worin das Suspensionsmittel 1 bis 5 % des Gewichts beträgt.

10. Produkt nach einem der vorhergehenden Ansprüche, worin das Gewichtsverhältnis von Suspensionsmittel zu Alkohol 1:1 bis 1:10 beträgt.

11. Produkt nach einem der vorhergehenden Ansprüche, das 0,5 bis 3 Gew.-% einer beruhigenden Flüssigkeit umfaßt, die weniger flüchtig als Isopropanol ist.

**12.** Produkt nach einem der vorhergehenden Ansprüche, worin das flüssige Treibmittel ein Kohlenwasserstoff ist, ausgewählt aus Pentan, Isopentan und Hexan oder Gemischen davon.

**13.** Produkt nach einem der Ansprüche 1 bis 11, worin das flüssige Treibmittel aus den Treibmitteln 11, 113 oder Gemischen davon ausgewählt ist.

**14.** Produkt nach einem der vorhergehenden Ansprüche, worin das flüssige Treibmittel 10 bis 60 % des Gewichts beträgt.

**15.** Produkt nach einem der vorhergehenden Ansprüche, worin das gasförmige Treibmittel ein Kohlenwasserstoff, ausgewählt aus Propan, Isobutan und n-Butan oder Gemischen davon, ist.

**16.** Produkt nach einem der Ansprüche 1 bis 14, worin das gasförmige Treibmittel ein Fluorkohlenstoff ist, ausgewählt aus Treibmittel C-318, Treibmittel 1141, Treibmittel 21 oder Treibmittel 114, oder Gemischen davon.

**17.** Produkt nach einem der Ansprüche 1 bis 14, worin das gasförmige Treibmittel ein Chlorfluorkohlenstoff ist, ausgewählt aus den Treibmitteln 22, 115, 12, 152a, 142b, oder Gemischen davon.

**18.** Produkt nach einem der vorhergehenden Ansprüche, worin das gasförmige Treibmittel 20 bis 80 % des Gewichts beträgt.

**19.** Produkt nach einem der vorhergehenden Ansprüche, das zusätzlich 0,001 bis 2 Gew.-% eines Parfüms umfaßt.

**20.** Verfahren zur Herstellung eines wasserfreien, Treibmittel enthaltenden, schweißhemmenden Produkts der Art, das einen feinzerteilten schweißhemmenden, in einer flüssigen Phase suspendierten Wirkstoff umfaßt, nach Anspruch 1, wobei das Verfahren die folgenden Schritte umfaßt:

(i) Vereinigen in einem geeigneten Behälter:

(a) 0,5 bis 8 Gew.-%, ausgedrückt in Form des Endprodukts, eines Suspensionsmittels, ausgewählt aus hydrophob behandelten Smektit-Tonen;

(b) 0,5 bis 25 Gew.-%, ausgedrückt in Form des Endprodukts, eines wasserfreien Alkohols, der weniger als 1 Gew.-% Wasser enthält und ausgewählt ist aus Ethanol, Isopropanol oder Gemischen davon; und

(c) 5 bis 70 Gew.-%, ausgedrückt in Form des Endprodukts, eines wasserfreien Treibmittels, das bei 20°C und einem Druck von 760 mm Hg flüssig ist, ausgewählt aus nichthalogenierten Kohlenwasserstoffen, Fluorkohlenstoffen, Chlorfluorkohlenstoffen, Methylenchlorid und Gemischen davon;

(ii) Unterziehen der Kombination einer starken Vermischung mit Scherwirkung für eine ausreichende Zeit, um eine einheitliche Dispersion des Suspensionsmittels zu erhalten; und

(iii) Mischen der Dispersion mit:

(a) 0,5 bis 20 Gew.-%, ausgedrückt in Form des Endprodukts, eines feinzerteilten, pulvrigen, schweißhemmenden Wirkstoffes, das einen maximalen Teilchendurchmesser von nicht größer als 100 μm hat, das im wesentlichen unlöslich in der flüssigen Phase des Produkts ist, und

(b) 5 bis 90 Gew.-%, ausgedrückt in Form des Endprodukts, eines wasserfreien, gasförmigen Treibmittels, ausgewählt aus nichthalogenierten Kohlenwasserstoffen, Fluorkohlenstoffen, Chlorfluorkohlenstoffen, Dimethylether, Kohlendioxid, Stickstoff oder Gemischen davon;

(c) einer weiteren Menge des Alkohol, wenn nötig, so daß das Endprodukt 2 bis 25 Gew.-% Alkohol enthält, um das schweißhemmende Produkt zu bilden.

**Revendications**

**1.** Un produit antiperspirant anhydre contenant un propuiseur convenant pour une application topique sur la peau, qui comprend :

a) de 0,5% à 20% en masse d'un agent antiperspirant en poudre finement divisée ayant un diamètre maximal de particule pas plus grand que 100μm qui est substantiellement insoluble dans la phase liquide du produit;

b) de 2 à 25% en masse d'alcool anhydre contenant moins de 1% en masse d'eau et choisi entre

16

EP 0 275 695 B1

l'éthanol, l'isopropanol et des mélanges de ceux-ci;

c) de 0,5% à 8% en masse d'un agent de mise en suspension choisi parmi les argiles traitées de manière hydrophobe;

d) de 0 à 3% en masse d'un liquide émollient qui est moins volatil que l'isopropanol;

e) de 0 à 70% en masse de propulseur anhydre qui est dans un état liquide à 20°C et 760mm de pression de Hg, choisi parmi les hydrocarbures non-halogénés, fluorocarbures, chlorofluorocarbures , chlorure de méthylène et des mélanges de ceux-ci; et

f) de 5 à 90% en masse de propulseur anhydre qui est dans un état gazeux à 20°C et 760mm de pression de Hg, choisi parmi les hydrocarbures non-halogénés, fluorocarbures, chlorofluorocarbures , éther diméthylique, dioxyde de carbone, azote et des mélanges de ceux-ci;

à condition que le rapport en masse de l'agent de mise en suspension à l'alcool anhydre dans le produit soit de 2:1 à 1:12.

2. Un produit selon la revendication 1, dans lequel l'agent antiperspirant est un halogénure basique contenant de l'aluminium.

3. Un produit selon la revendication 1 ou 2, dans lequel l'agent antiperspirant est choisi parmi le chlorure d'aluminium, le sulfate d'aluminium, le chlorohydrate d'aluminium, des complexes basiques glycine chlorure d'aluminium, des complexes basiques urée chlorure d'aluminium, ou des mélanges de ceux-ci.

4. Un produit selon la revendication 1, 2 ou 3 dans lequel l'agent antiperspirant est choisi parmi des complexes d'urée ou de glycine et de chlorhydrate d'aluminium, ou des mélanges de ceux-ci.

5. Un produit selon l'une quelconque des revendications précédentes, dans lequel l'agent antiperspirant représente de 1 à 15% de la masse.

6. Un produit selon l'une quelconque des revendications précédentes, dans lequel l'agent antiperspirant représente de 2 à 12% de la masse.

7. Un produit selon l'une quelconque des revendications précédentes, dans lequel l'alcool représente de 5 à 20% de la masse.

8. Un produit selon l'une quelconque des revendications précédentes, dans lequel l'argile est une argile smectique.

9. Un produit selon l'une quelconque des revendications précédentes, dans lequel l'agent de mise en suspension représente de 1 à 5% de la masse.

10. Un produit selon l'une quelconque des revendications précédentes, dans lequel le rapport en masse de l'agent de mise en suspension à l'alcool va de 1:1 à 1:10.

11. Un produit selon l'une quelconque des revendications précédentes, qui comprend de 0,5 à 3% en masse d'un liquide émollient qui est moins volatil que l'isopropanol.

12. Un produit selon l'une quelconque des revendications précédentes, dans lequel le propulseur liquide est un hydrocarbure choisi parmi le pentane, l'isopentane, et l'hexane ou des mélanges de ceux-ci.

13. Un produit selon l'une quelconque des revendications 1 à 11, dans lequel le liquide propulseur est choisi parmi les Propellants 11, 113 ou des mélanges de ceux-ci.

14. Un produit selon l'une quelconque des revendications précédentes, dans lequel le liquide propulseur constitue de 10 à 60% de la masse.

15. Un produit selon l'une quelconque des revendications précédentes, dans lequel le propulseur gazeux est un hydrocarbure choisi parmi le propane, l'isobutane, et le n-butane ou des mélanges de ceux-ci.

16. Un produit selon l'une quelconque des revendications 1 à 14, dans lequel le propulseur gazeux est un fluorocarbure choisi parmi le Propellant C-318, le Propellant 1141, le Propellant 21 ou le Propellant 114,

17

ou des mélanges de ceux-ci.

17. Un produit selon l'une quelconque des revendications 1 à 14, dans lequel le propulseur gazeux est un chlorofluorocarbure choisi parmi les Propellants 22, 115, 12, 152a, 142b, ou des mélanges de ceux-ci.

18. Un produit selon l'une quelconque des revendications précédentes, dans lequel le gaz propulseur constitue de 20 à 80% de la masse.

19. Un produit selon l'une quelconque des revendications précédentes, qui comprend de plus de 0,001 à 2% en masse d'un parfum.

20. Un procédé pour préparer un produit antiperspirant anhydre contenant un propulseur du type comprenant un actif antiperspirant finement divisé mis en suspension dans une phase liquide, en conformité avec la revendication 1, lequel procédé comprend les étapes de :
    (i) combiner dans un récipient approprié :
        (a) de 0,5 à 8% en masse, relativement au produit final, d'un agent de mise en suspension choisi parmi des argiles smectiques traitées de manière hydrophobe;
        (b) une quantité entre 0,5 et 25% en masse, relativement au produit final, d'alcool anhydre contenant moins de 1% en masse d'eau et choisi entre l'éthanol, l'isopropanol et des mélanges de ceux-ci; et
        (c) entre 5 et 70% en masse, relativement au produit final, d'un propulseur anhydre qui est un liquide à 20°C et 760mmHg, choisi parmi des hydrocarbures non-halogénés, des fluorocarbures, des chlorofluorocarbures, le chlorure de méthylène et des mélanges de ceux-ci;
    (ii) soumettre la combinaison à un mixtionnage avec un fort taux de cisaillement pendant une période de temps suffisante pour accomplir une dispersion uniforme de l'agent de mise en suspension; et
    (iii) mélanger la dispersion avec :
        (a) de 0,5 à 20% en masse, relativement au produit final, d'un agent antiperspirant en poudre finement divisée, ayant un diamètre maximum de particule pas plus grand que 100μm, qui est substantiellement insoluble dans la phase liquide du produit, et
        (b) de 5 à 90% en poids, relativement au produit final, d'un propulseur gazeux anhydre, choisi parmi des hydrocarbures non-halogénés, des fluorocarbures, des chlorofluorocarbures, le diméthyle éther, le dioxyde de carbone, l'azote et des mélanges de ceux-ci;
        (c) une quantité supplémentaire si nécessaire de l'alcool afin que le produit final contienne de 2 à 25% en masse d'alcool;
    pour former le produit antiperspirant.